# EUROPEAN PATENT APPLICATION

(11) **EP 3 138 567 A1**
(43) Date of publication of application: **08.03.2017**
(21) Application number: 15786347.3
(22) Date of filing: 28.04.2015
(51) Int. Cl.: A61K 31/522, A61K 33/14, A61K 31/194, A61P 11/08

(54) **PHARMACEUTICAL COMPOSITION FOR TREATING CHRONIC OBSTRUCTIVE PULMONARY DISEASES**

(30) Priority: 29.04.2014 UA 1404623
(71) Applicant: Gumeniuk, Mykola Ivanovych, Kiev 03110 (UA)
(72) Inventor: DERKACH, Nataliia Mykolaivna, Kiev 03110 (UA)
(74) Representative: Anohins, Vladimirs
(86) International application number: PCT/UA2015/000038
(87) International publication number: WO 2015/167416

(57) **Abstract**

The technical solution is related to preparations for treatment of chronic obstructive pulmonary diseases, namely, broncholytic drugs.

Pharmaceutical composition for treatment of chronic obstructive pulmonary diseases comprises theophylline, water, potassium chloride, magnesium chloride, and succinic acid or its salt.

The technical solution expands the assortment of drugs, and the theophylline-based pharmaceutical composition according to the technical solution when used in the treatment of chronic obstructive pulmonary diseases causes less adverse reactions, in particular, less arhythmogenic effect.

## Description

### Field of invention

The technical solution is related to preparations for treatment of chronic obstructive pulmonary diseases, namely, to broncholytic drugs.

### Prior art

Chronic obstructive pulmonary diseases (hereinafter abbreviated as COPD) are chronic pathology with progressive respiratory obstruction and development of pulmonary hypertension. The notion of COPD unites several diseases with inherently inhomogeneous clinical conditions, in particular:
- Pulmonary emphysema;
- Chronic obstructive bronchitis;
- Chronic obstructive bronchitis in conjunction with pulmonary emphysema;
- Bronchial asthma;
- Bronchial asthma in conjunction with chronic obstructive bronchitis;
- Obstructive nocturnal apnea;
- Multiple bronchiectasis;
- Multiple bronchiectasis in conjunction with chronic obstructive bronchitis.

In contemporary society, chronic obstructive pulmonary disease (COPD), along with arterial hypertension, ischemic heart disease and diabetes mellitus comprise leading group of chronic diseases: they account for more than 30% of all human pathologies.

The World Health Organization (WHO) puts COPD to the group of diseases having high level of social burden, because of its wide incidence both in developed and in developing countries. The forecast composed by WHO experts up to year 2020 testifies to the effect that COPD will become not only one of the most prevalent forms of human pathology, but also one of the leading mortality causes, while there is expected a decrease in mortality rates for myocardial infarction, oncological diseases etc.

The most frequent concomitant diseases with COPD include cachexia, hypotrophy and atrophy of skeletal muscles, arterial hypertension, ischemic heart disease, heart failure, vasculopathies of the pulmonary circulation, respiratory infectious diseases and oncological diseases.

The prognosis is most unfavorable for COPD in conjunction with a group of cardiovascular diseases. Patients suffering from severe forms of COPD belong to a group with a high risk of sudden death. One of the reasons that may cause sudden death is cardiac rhythm disturbance. Supraventricular and ventricular forms of the cardiac rhythm disturbances are clinical problems quite widespread in COPD patients. However, there exist substantial variations in data presented in literature. Large fluctuations in data on arrhythmias in COPD patients are accounted for by different populations of the patients participating in the studies; different stages of disease and degree of clinical manifestations of COPD, as well as methodological conditions for EKG registration and monitoring. Undoubtedly, significant role belongs to concurrent ischemic heart disease and ventricular failure present.

In case of COPD, another major reason for the onset of arrhythmic events is related to administration of certain drugs, in particular, theophylline.

Theophylline is prescribed for bronchoobstructive syndrome of any genesis, such as bronchial asthma, chronic obstructive bronchitis, pulmonary emphysema, pulmonary hypertension, obstructive nocturnal apnea, and the like.

Among considerable diversity of drugs historically administered to COPD patients, the most investigated are arrhythmogenic effects of theophylline and its derivatives. Administration of theophylline and its derivatives has been associated with such arrhythmias as sinus tachycardia, premature atrial contractions, supraventricular tachycardia, atrial fibrillation, unifocal and multifocal atrial tachycardia, ventricular arrhythmias. The emergence of both atrial and ventricular arrhythmias is directly related to the serum concentration of theophylline. Toxic effects of theophylline may be facilitated by smoking, administration of macrolides, and antihistamine drugs.

Of certain importance in the emergence of arrhythmogenic effects of theophylline are age, concomitant diseases, such as ischemic heart disease, liver diseases, and some others. In the studies conducted by Bittar G., H.S. Friedman (The arrhythmogenicity of theophylline. A multivariate analysis of clinical determinants. Chest 1991 Jun; 99 (6): 1415-20), authors have investigated the relation between serum concentration of theophylline and development of arrhythmic events in COPD patients admitted to clinics in the acute stage of the disease. The study was carried out on a group of 100 patients. Many factors involved in the development of arrhythmias have been taken into account, such as digoxin concentration, potassium concentration and a lot of other parameters. Authors have reached a conclusion that arrhythmogenic effects are caused primarily by theophylline administration. Heart arrhythmias, some of which have been classified as life-threatening (for example, ventricular tachycardia, polytopic ventricular extrasystole and other forms), have developed even against the background of recommended therapeutical concentration of theophylline.

The reasons of toxic effects of theophylline in COPD treatment are explained by two factors, namely, dose and duration of administration. With blood theophylline levels at 20-30 µg/ml adverse reactions may be observed on the side of cardiovascular system, while at concentration in excess of 35 mg/l risk of developing severe arrhythmia increases, together with onset of CNS disorders, hyperglycemia, hypokalemia, and hypomagnesemia. Potassium and magnesium deficiency in COPD patients is associated with increase in the number of arrhythmia cases. At present there is no doubt about the necessity of medicamental correction of electrolyte disorders (hypomagnesemia and hypokalemia) in COPD patients in the course of therapy with theophylline-based preparations.

There is known a pharmaceutical composition comprising theophylline, water, and auixiliary components, such as sodium acetate trihydrate and sodium hydroxide (specification to a patent of Ukraine for invention No. 29558, published on 15.11.2000). The pharmaceutical composition is intended, in particular, for treatment of bronchial asthma, but may be used in other COPD-related diseases. The components of known pharmaceutical composition, such as sodium acetate trihydrate and sodium hydroxide, are used to increase theophylline solubility in water and to adjust pH of the pharmaceutical composition. A shortcoming of known pharmaceutical composition is undesirable adverse arrhythmogenic effect inherent to theophylline as described above.

### Disclosure of the invention

The problem to be solved by the technical solution is diversification of drugs choice and development of theophylline-based pharmaceutical composition for treatment of chronic obstructive pulmonary diseases, which would produce less adverse reactions, in particular, less arrhythmogenic effect.

The problem is solved by the pharmaceutical composition for treatment of chronic obstructive pulmonary diseases comprising theophylline, water, potassium chloride, magnesium chloride, succinic acid or its salt, at following components ratio, mg/ml:

| | |
|---|---|
| theophylline | 1.5 - 2.5 |
| potassium chloride | 0.2 - 0.4 |
| magnesium chloride | 0.1 - 0.3 |
| succinic acid or its salt | 11 - 18 |
| water | up to 1 ml |

Moreover, pharmaceutical composition for treatment of chronic obstructive pulmonary diseases may comprise theophylline, water, potassium chloride, magnesium chloride, succinic acid or its salt, preferably at following components ratio, mg/ml:

| | |
|---|---|
| theophylline | 1.9 - 2.1 |
| potassium chloride | 0.27 - 0.33 |
| magnesium chloride | 0.17 - 0.23 |
| succinic acid or its salt | 14 - 16 |
| water | up to 1 ml |

Moreover, chronic obstructive pulmonary diseases include pulmonary emphysema, or chronic obstructive bronchitis, or chronic obstructive bronchitis in conjunction with pulmonary emphysema, or bronchial asthma, or bronchial asthma in conjunction with chronic obstructive bronchitis, or obstructive nocturnal apnea.

Moreover, the succinic acid salt may be disodium succinate.

Control of hypoxemia, hypokalemia, and hypomagnesemia is an importatnt part of treatment regimen for COPD patients, allowing the patient not only to resolve the acute condition but also to prevent undesirable adverse reactions on the part of considerable group of drugs, in particular, theophylline. Treatment regimens of cardiac arrhythmias in COPD patients have quite a lot of specifics. The important place in the treatment is assigned to the correction of hypokalemia and hypomagnesemia.

The components of the claimed pharmaceutical composition, such as potassium chloride and magnesium chloride, prevent development of hypokalemia and hypomagnesemia during theophylline administration in treatment of COPD patients. Antiarrhythmic effect of magnesium occur by its influence on the transport of sodium, potassium, and calcium ions participating in shaping the action potential of cardiac muscles. Magnesium facilitates potassium uptake and provides for its optimal intracellular levels. Total potassium and magnesium deficiency during theophylline treatment results iin hypokalemia, which is resistant to treatment with potassium-containing preparations administered in addition to theophylline treatment.

Simultaneous inclusion of potassium chloride and magnesium chloride into the claimed pharmaceutical composition in amounts claimed ensures necessary level of calcium and magnesium ions and correspondingly prevents development of hypokalemia and hypomagnesemia in the course of treatment, which in its turn leads to decrease in the development of cardiac rhythm abnormalities and correspondingly reduces incidence of arrhythmia cases.

In clinical practice, succinic acid preparations are used in the therapy of cardiovascular pathology, in impairments of brain circulation, to stimulate digestive juice secretion, as antidote in arsenic, lead, and mercury intoxications, and in various other situations. Succinic acid is used in complex treatment of ischemic heart disease. Its effects are caused mainly by activation of enzymes oxidation, including those participating in oxygen supply to mitochondria. Cardiac metabolism normalization and restoration of coronary blood flow ensure antiarrhythmic effects of succinic acid and prevent the development of hypoxemia.

Inclusion of succinic acid or its salt into the claimed pharmaceutical composition promotes cardiac muscle tone in COPD patients with cardiovascular diseases, heighten energy balance of the heart, increases exercise tolerance of cardiac muscle, while the presence in the claimed pharmaceutical composition of succinic acid or its salt together with potassium and magnesium salts provides for synergetic antiarrhythmic effect.

The amount claimed of succinic acid or its salt, and that of potassium chloride and magnesium chloride in the claimed pharmaceutical composition is sufficient to achieve synergetic antiarrhythmic effect.

Theophylline refers to theophylline in any form, including salt form, or crystalline hydrate form, for example, monohydrate form of theophylline.

Possibility of the preparation of pharmaceutical composition according to the technical solution is further demonstrated by Examples 1 to 6.

### Example 1.

To obtain a batch of the pharmaceutical composition of required volume, the following main operations are performed sequentially:
- dissolving potassium chloride by its addition to a portion of water at a temperature of 25-45 °C having volume less than that required for a batch of the pharmaceutical composition, and mixing to complete dissolution of potassium chloride;
- dissolving magnesium chloride in the above obtained solution by adding magnesium chloride to the solution and mixing to complete dissolution of magnesium chloride;
- dissolving succinic acid or its salt in the above obtained solution by adding succinic acid or its salt to the solution and mixing to complete dissolution of succinic acid or disodium salt of succinic acid;
- dissolving theophylline in the above obtained solution by adding theophylline to the solution and mixing to complete dissolution of theophylline;
- cooling down the above obtained solution to 20 °C;
- preparing the solution of the pharmaceutical composition by adding a portion of water to the above obtained solution to bring the volume of the solution up to the required volume of the pharmaceutical composition, and filtering through the filtration manifold with a set of filters with pore sizes of 1 to 0.22 micron.

To prepare 1 ml of the pharmaceutical composition, 0.2 mg of potassium chloride is added to 0.4 ml portion of water at 25 °C and agitated to complete dissolution of potassium chloride.

0.1 mg of magnesium chloride is added to the solution and agitated to complete dissolution of magnesium chloride.

11 mg of succinic acid is added to the solution and agitated to complete dissolution of succinic acid.

1,5 mg of theophylline is added to the solution and agitated to complete dissolution of theophylline.

The solution is cooled down to 20 °C, and a portion of water is added to bring the volume of the solution up to 1 ml. The solution is filtered through the filtration manifold with a set of filters with pore sizes of 1 to 0.22 micron.

### Example 2.

In order to prepare a portion of the pharmaceutical composition of required volume main operations are performed as described in general in Example 1.

To prepare 1 ml of the pharmaceutical composition, 0.24 mg of potassium chloride is added to 0.45 ml portion of water at 30 °C and agitated to complete dissolution of potassium chloride.

0.14 mg of magnesium chloride is added to the solution and agitated to complete dissolution of magnesium chloride.

13 mg of disodium salt of succinic acid is added to the solution and agitated to complete dissolution of disodium salt of succinic acid.

1.7 mg of theophylline monohydrate is added to the solution and agitated to complete dissolution of theophylline monohydrate.

The solution is cooled down to 20 °C, and a portion of water is added to bring the volume of the solution up to 1 ml, and then filtered through the filtration manifold with a set of filters with pore sizes of 1 to 0.22 micron.

### Example 3.

In order to prepare a portion of the pharmaceutical composition of required volume main operations are performed as described in general in Example 1.

To prepare 1 ml of the pharmaceutical composition 0.28 mg of potassium chloride is added to 0.5 ml portion of water at 30 °C and agitated to complete dissolution of potassium chloride.

0.18 mg of magnesium chloride is added to the solution and agitated to complete dissolution of magnesium chloride.

15 mg of succinic acid is added to the solution and agitated to complete dissolution of succinic acid.

1.9 mg of theophylline is added to the solution and agitated to complete dissolution of theophylline.

The solution is cooled down to 20 °C, and a portion of water is added to bring the volume of the solution up to 1 ml, and then filtered through the filtration manifold with a set of filters with pore sizes of 1 to 0.22 micron.

### Example 4.

In order to prepare a portion of the pharmaceutical composition of required volume, operations are performed as described in general in Example 1.

To prepare 1 ml of the pharmaceutical composition, 0.32 mg of potassium chloride is added to 0.55 ml portion of water at 35 °C and agitated to complete dissolution of potassium chloride.

0.22 mg of magnesium chloride is added to the solution and agitated to complete dissolution of magnesium chloride.

17 mg of disodium salt of succinic acid is added to the solution and agitated to complete dissolution of disodium salt of succinic acid.

2.1 mg of theophylline monohydrate is added to the solution and agitated to complete dissolution of theophylline monohydrate.

The solution is cooled down to 20 °C and a portion of water is added to bring the volume of the solution up to 1 ml, and then filtered through the filtration manifold with a set of filters with pore sizes of 1 to 0.22 micron.

### Example 5.

In order to prepare a portion of the pharmaceutical composition of required volume, operations are performed as described in general in Example 1.

To prepare 1 ml of the pharmaceutical composition, 0.36 mg of potassium chloride is added to 0.6 ml portion of water at 40 °C and agitated to complete dissolution of potassium chloride.

0.26 mg of magnesium chloride is added to the solution and agitated to complete dissolution of magnesium chloride.

19 mg of succinic acid is added to the solution and agitated to complete dissolution of succinic acid.

2.3 mg of theophylline is added to the solution and agitated to complete dissolution of theophylline.

The solution is cooled down to 20 °C and a portion of water is added to bring the volume of the solution up to 1 ml, and then filtered through the filtration manifold with a set of filters with pore sizes of 1 to 0.22 micron.

### Example 6.

In order to prepare a portion of the pharmaceutical composition of required volume, operations are performed as described in general in Example 1.

To prepare 1 ml of the pharmaceutical composition, 0.4 mg of potassium chloride is added to 0.65 ml portion of water at 45 °C and agitated to complete dissolution of potassium chloride.

0.3 mg of magnesium chloride is added to the solution and agitated to complete dissolution of magnesium chloride.

21 mg of disodium salt of succinic acid is added to the solution and agitated to complete dissolution of disodium salt of succinic acid.

2.5 mg of theophylline monohydrate is added to the solution and agitated to complete dissolution of theophylline monohydrate.

The solution is cooled down to 20 °C and a portion of water is added to bring the volume of the solution up to 1 ml. The solution is filtered through the filtration manifold with a set of filters with pore sizes of 1 to 0.22 micron.

To study the efficiency of the claimed pharmaceutical composition and its tolerability, a comparative open randomized study has been carried out in two groups of patients with bronchial asthma.

Patients in the main group were administered on the background of basic therapy the pharmaceutical composition claimed, while patients in the control group were administered on the background of basic therapy a preparation comprising only theophylline (described in patent of Ukraine for the invention No. 29558). The treatment continued for 14 days.

The studies have included 78 patients - male and female, aged from 18 to 70 years, having the diagnosis: bronchial asthma of moderate severity as regards inclusion criteria. Statistical data on patients in groups, duration of their disease, and condition of their cardiovascular system are presented in Tables 1-3.

**Table 1**

| Sex distribution of patients in the main and control groups | | | | | | |
|---|---|---|---|---|---|---|
| **Sex** | **Main group** | | **Control group** | | **Total** | |
| | **n** | **%** | **n** | **%** | **n** | **%** |
| Male | 15 | 38.7 | 14 | 34.7 | 29 | 36.7 |
| Female | 24 | 61.3 | 25 | 65.3 | 49 | 63.3 |
| Total | 39 | 100.0 | 39 | 100.0 | 78 | 100.0 |

Registration of electrocardiographic data for two groups of patients during treatment had been performed three times - on Day 1 of treatment, on Day 4 of treatment and on Day 14 - last day of treatment. Electrocardiographic data were used to determine adverse reactions, such as arrhythmia, and data of observations - to determine such adverse reactions as headache and tremor of limbs. The results of the studies are presented in Tables 4 to 7.

It is seen from the results of these studies that most patients after administration of the claimed pharmaceutical composition have demonstrated good tolerance of the claimed pharmaceutical composition. Some patients displayed adverse reactions in the form of cardiac rhythm disorder events, headache, and tremor of limbs. All the adverse reactions observed were of moderate or small intensity, and no treatment cessation was required. Frequency and intensity of adverse reactions in patients of the main group were different from those of adverse reactions in patients of the control group - patients in the main group have displayed substantially less frequency of arrhythmia cases (7.9% incidence), than patients in the control group (23.7% incidence).

**Table 2**

| Distribution of test subjects by disease duration, % | | | | |
|---|---|---|---|---|
| **Duration of disease, years** | **Main group** | | **Control group** | |
| | **n** | **%** | **n** | **%** |
| up to 5 years | 2 | 4.0 | 2 | 4.0 |
| from 5 to 10 years | 5 | 12.0 | 6 | 16.0 |
| from 11 to 15 years | 20 | 52.0 | 20 | 50.7 |
| more than 15 years | 12 | 32.0 | 11 | 29.3 |
| Total | 39 | 100.0 | 39 | 100.0 |

**Table 3**

| Distribution of the initial state of the cardiovascular system of patients by electrocardiographic (ECG) data | | | | |
|---|---|---|---|---|
| **ECG data** | **Main group** | | **Control group** | |
| | **n** | **%** | **n** | **%** |
| Sinus tachycardia (heart rate >80 beats per minute) | 38 | 100.0 | 38 | 100.0 |
| Infrequent single supraventricular extrasystole | 20 | 5.3 | 2 | 4.0 |
| Infrequent single ventricular extrasystole | 10 | 2.7 | 5 | 4.0 |
| Total | 38 | 100.0 | 38 | 100.0 |

The results of the studies demonstrate substantial antiarrhythmic effect of the claimed pharmaceutical composition (number of arrhythmia cases reduced by 66.6% in comparison with the control group) and pronounced effect of decreasing the total number of adverse reactions (total number of adverse reactions cases reduced by 52.9% in comparison with the control group).

To determine the contribution of each component of the claimed pharmaceutical composition to the effect achieved, additional studies have been carried out with two groups of patients. Number of patients in these groups was the same as in the first study, statistical data of patients and state of their cardiovascular system were quite similar to those in the first study.

**Table 4**

| Distribution of patients in the main group by electrocardiographic (ECG) data | | | | |
|---|---|---|---|---|
| **ECG data** | | **Treatment day** | | |
| | | **1** | **4** | **14** |
| Sinus tachycardia (heart rate >80 beats per minute) | present | 1 | 2 | 2 |
| | absent | 36 | 35 | 33 |
| | **total** | 38 | 38 | 38 |
| Supraventricular extrasystole | present | 2 | 3 | 1 |
| | absent | 34 | 35 | 35 |
| | **total** | 38 | 38 | 38 |
| Ventricular extrasystole | present | 2 | 1 | - |
| | absent | 36 | 37 | 38 |
| | **total** | 38 | 38 | 38 |

**Table 5**

| Distribution of patients in the control group by electrocardiographic (ECG) data | | | | |
|---|---|---|---|---|
| **ECG data** | | **Treatment day** | | |
| | | **1** | **4** | **14** |
| Sinus tachycardia (heart rate >80beats per minute) | present | 2 | 4 | 4 |
| | absent | 36 | 34 | 34 |
| | **total** | 38 | 38 | 38 |
| Supraventricular extrasystole | present | 1 | 2 | 3 |
| | absent | 34 | 34 | 34 |
| | **total** | 38 | 38 | 38 |
| Ventricular extrasystole | present | 1 | 1 | 2 |
| | absent | 37 | 37 | 36 |
| | **total** | 38 | 38 | 38 |

The studies have been conducted similar to the first study, patients of the group 1 were administered pharmaceutical composition comprising theophylline, potassium chloride, and magnesium chloride on the background of basic therapy, while patients of the group 2 were administered pharmaceutical composition comprising theophylline and succinic acid on the background of basic therapy. Concentrations of theophylline, potassium chloride, magnesium chloride, and succinic acid were similar to concentrations of the claimed pharmaceutical composition in the first study. Treatment continued for 14 days.

The results of the second study are presented in Table 8.

**Table 6**

| Distribution of patients by character and frequency of adverse reactions | | | | |
|---|---|---|---|---|
| **Adverse reactions** | **Main group** | | **Control group** | |
| | **Number** | **Fraction, %** | **Number** | **Fraction, %** |
| Arrhythmia | 3 | 7.9 | 9 | 23.7 |
| Headache | 3 | 7.9 | 4 | 10.5 |
| Tremor of limbs | 2 | 5.3 | 4 | 10.5 |
| Total patients with adverse reactions | 8 | 23.7 | 17 | 44.7 |

**Table 7**

| The results of the tolerability analysis | | | | | |
|---|---|---|---|---|---|
| **Parameter** | **Category** | **Main group** | | **Control group** | |
| | | **n** | **%** | **n** | **%** |
| General tolerability | Good | 28 | 73.7 | 27 | 71 |
| | Satisfactory | 10 | 26.3 | 11 | 29 |
| | Unsatisfactory | - | - | - | - |
| | Total | 38 | 100 | 38 | 100 |

Comparison of the results of first and second studies indicates that the addition of succinic acid to theophylline has insufficient effect both on decrease of arrhythmia cases (number of arrhythmia cases decreased by 11.1% in comparison with the control group), and on decrease of total number of adverse reactions (total number of cases of adverse reactions decreased by 11.8% in comparison with the control group).

The comparison of the results of first and second studies indicates also that the addition of potassium chloride and magnesium chloride to theophylline has more pronounced effect both on decrease of arrhythmia cases (number of arrhythmia cases decreased by 33.3% in comparison with the control group), and on decrease of total number of adverse reactions (total number of cases of adverse reactions decreased by 29.4% in comparison with the control group).

The results of first and second studies demonstrate that components of the claimed pharmaceutical composition, such as potassium chloride, magnesium chloride, and succinic acid or its salt, create together an unexpected synergetic effect. The sum of percentages of decrease in the number of arrhythmia cases for the pharmaceutical composition comprising theophylline, potassium chloride, and magnesium chloride, and that for the pharmaceutical composition comprising theophylline and succinic acid, amounts to 33.3 + 11.1 = 44.4 %, which is less than the percentage of decrease in the number of arrhythmia cases for the claimed pharmaceutical composition (66.6%). The sum of percentages of decrease in total number of adverse reactions for the pharmaceutical composition comprising theophylline, potassium chloride, and magnesium chloride, and that of the adverse reactions for the pharmaceutical composition comprising theophylline and succinic acid amounts to 29.4 + 11.8 = 41.2%, is less than the percentage of decrease in total number of adverse reactions cases for the claimed pharmaceutical composition (52.9%). In pharmacology, a case when the value of the effect of using combination of two substances is greater than the sum of effects of using the substances separately is called potentiation. Potentiation is one of the kinds of synergism in pharmacology. The concomitant use of certain components, such as potassium chloride, magnesium chloride, and succinic acid or its salt, in the claimed pharmaceutical composition, provides an effect of potentiation, and correspondingly they display together a synergetic effect, which is unpredictable.

**Table 8**

| Distribution of patients by character and frequency of adverse reactions | | | | |
|---|---|---|---|---|
| **Adverse reactions** | **Group 1** | | **Group 2** | |
| | **Number** | **Fraction, %** | **Number** | **Fraction, %** |
| Arrhythmia | 6 | 15.8 | 8 | 21.1 |
| Headache | 3 | 7.9 | 4 | 10.5 |
| Tremor of limbs | 3 | 7.9 | 3 | 7.9 |
| Total patients with adverse reactions | 12 | 31.6 | 15 | 39.5 |

Thus, it can be concluded that the technical solution expands the assortment of drugs, and the theophylline-based pharmaceutical composition according to the technical solution when used in the treatment of chronic obstructive pulmonary diseases causes less adverse reactions, in particular, less arhythmogenic effect.

The examples of the embodiments provided herein illustrate the technical solution without limiting it.

## Claims

1. A pharmaceutical composition for treatment of chronic obstructive pulmonary diseases, comprising theophylline and water, **characterized in that** the composition further comprises potassium chloride, magnesium chloride, succinic acid or its salt, at following components ratio, mg/ml:
| | |
|---|---|
| theophylline | 1.5 - 2.5 |
| potassium chloride | 0.2 - 0.4 |
| magnesium chloride | 0.1 - 0.3 |
| succinic acid or its salt | 11 - 18 |
| water | up to 1 ml. |

2. The pharmaceutical composition for treatment of chronic obstructive pulmonary diseases according to claim 1, **characterized in that** the composition comprises theophylline, water, potassium chloride, magnesium chloride, succinic acid or its salt, preferably at following components ratio, mg/ml:
| | |
|---|---|
| theophylline | 1.9 - 2.1 |
| potassium chloride | 0.27 - 0.33 |
| magnesium chloride | 0.17 - 0.23 |
| succinic acid or its salt | 14 - 16 |
| water | up to 1 ml. |

3. The pharmaceutical composition for treatment of chronic obstructive pulmonary diseases according to claim 1, **characterized in that** the chronic obstructive pulmonary disease comprises pulmonary emphysema, or chronic obstructive bronchitis, or chronic obstructive bronchitis in conjunction with pulmonary emphysema, or bronchial asthma, or bronchial asthma in conjunction with chronic obstructive bronchitis, or obstructive nocturnal apnea.

4. The pharmaceutical composition for treatment of chronic obstructive pulmonary diseases according to claim 2, **characterized in that** the chronic obstructive pulmonary disease comprises pulmonary emphysema, or chronic obstructive bronchitis, or chronic obstructive bronchitis in conjunction with pulmonary emphysema, or bronchial asthma, or bronchial asthma in conjunction with chronic obstructive bronchitis, or obstructive nocturnal apnea.

5. Pharmaceutical composition for treatment of chronic obstructive pulmonary diseases according to any of claims 1 and 2, **characterized in that** the salt of succinic acid is disodium succinate.
